(19) Europäisches Patentamt
European Patent Office
Office européen des brevets

(11) Veröffentlichungsnummer: **0 519 434 A2**

(12) # EUROPÄISCHE PATENTANMELDUNG

(21) Anmeldenummer: **92110245.5**

(22) Anmeldetag: **17.06.92**

(51) Int. Cl.⁵: **C07D 487/04**, A61K 31/495, //(C07D487/04,249:00,241:00)

(30) Priorität: **21.06.91 DE 4120494**

(43) Veröffentlichungstag der Anmeldung: **23.12.92 Patentblatt 92/52**

(84) Benannte Vertragsstaaten: **AT BE CH DE DK ES FR GB GR IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Heitsch, Holger, Dr.**
**Martin-Wohmann-Strasse 27**
**W-6238 Hofheim am Taunus(DE)**
Erfinder: **Henning, Rainer, Dr.**
**Im Höhlchen 16**
**W-6234 Hattersheim/M.(DE)**
Erfinder: **Urbach, Hansjörg, Dr.**
**Le Lavandoustrasse 41**
**W-6242 Kronberg/Ts.(DE)**
Erfinder: **Ruppert, Dieter, Dr.**
**Schreyerstrasse 30**
**W-6242 Kronberg/Ts.(DE)**
Erfinder: **Linz, Wolfgang, Dr.**
**Huxelrebenweg 54**
**W-6500 Mainz 42(DE)**

(54) **Heterocyclische Verbindungen mit reninhemmenden Eigenschaften, Verfahren zu ihrer Herstellung und ihre Verwendung.**

(57)

Heterocyclische Verbindungen der Formel (I),

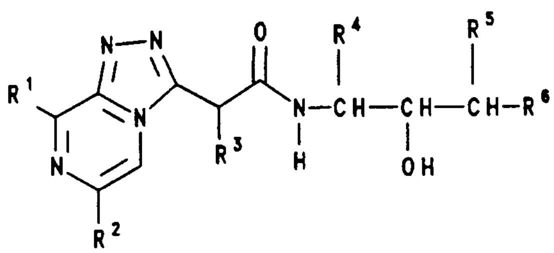

(I)

in welcher die Reste folgende Bedeutungen haben:

- $R^1$ z.B. Propyl und Isobutyl
- $R^2$ z.B. Phenyl oder Pyridyl
- $R^3$ z.B. Wasserstoff,
- $R^4$ z.B. Cyclohexylmethyl
- $R^5$ z.B Wasserstoff oder Hydroxy
- $R^6$ ein Rest der Formel (II),

$(CH_2)_s$-$CHR^7$-Z (II)

EP 0 519 434 A2

wobei
$R^7$ z.B Wasserstoff,
Z für einen 5- bis 7-gliedrigen heterocyclischen Ring und
s 0, 1, 2, 3 oder 4 bedeutet;
sind potente Renin-hemmende Verbindungen.

Aus EP-A 370 454, EP-A 373 497, EP-A 394 853 und EP-A 417 698 sind C-terminal heterocyclisch substituierte Aminodiolderivate mit renininhibitorischer Wirkung bekannt. In EP-A 369 743 sowie J. Med. Chem. 33(1990), 2326-2334, J. Med. Chem. 33(1990), 2335-2342 und J. Med. Chem. 34 (1991), 151-157 werden Alkylalkohol- und Statin-Derivate beschrieben, die zwei über eine Alkylamino-Brücke verbundene heterocyclische Systeme enthalten.

Überraschenderweise wurde nun gefunden, daß Verbindungen, die zwei über eine Alkylamino-Brücke verbundene heterocyclische Systeme enthalten, außergewöhnlich wirksame und hochspezifische Reninhemmer sind, die eine wesentlich verbesserte Wirkung in vivo und eine erheblich gesteigerte Resorption im Magen-Darmtrakt aufweisen.

Die Erfindung betrifft daher Verbindungen der Formel I

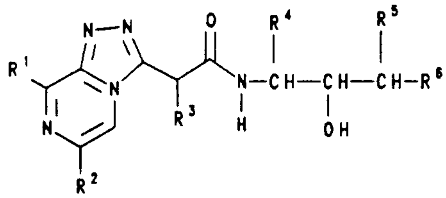

(I)

in welcher die Reste folgende Bedeutungen haben:

- $R^1$ ($C_1$-$C_8$)-Alkyl oder Phenyl;
- $R^2$ Phenyl oder Pyridyl, wobei der Pyridylrest gegebenenfalls einen ($C_1$-$C_4$)-Alkylsubstituenten trägt;
- $R^3$ Wasserstoff oder eine Gruppe der Formel Q-A, in welcher Q steht für Pyridyl, Imidazolyl, Thiazolyl und Pyrazolyl, und A Methylen oder Ethylen ist
- $R^4$ Wasserstoff, ($C_1$-$C_{10}$)-Alkyl, ($C_3$-$C_8$)-Cycloalkyl, ($C_3$-$C_8$)-Cycloalkyl-($C_1$-$C_4$)-alkyl, ($C_6$-$C_{12}$)-Aryl oder ($C_6$-$C_{12}$)-Aryl-($C_1$-$C_4$)-alkyl;
- $R^5$ Wasserstoff, ($C_1$-$C_{10}$)-Alkyl, ($C_6$-$C_{12}$)-Aryl, ($C_6$-$C_{12}$)-Aryl-($C_1$-$C_4$)-alkyl, Hydroxy oder Amino;
- $R^6$ ein Rest der Formel (II),

$(CH_2)_s$-$CHR^7$-Z (II)

wobei

$R^7$ für Wasserstoff, ($C_1$-$C_7$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht, und

Z für einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann, und der durch einen oder zwei gleiche oder verschiedene Reste aus der Reihe ($C_1$-$C_4$)-Alkyl, Allyl, ($C_1$-$C_4$)-Alkoxy, Hydroxy, Halogen, Amino, Mono- oder Di-($C_1$-$C_4$)-alkylamino und $CF_3$ substituiert sein kann; und wobei

s 0, 1, 2, 3 oder 4 bedeutet; sowie deren physiologisch verträgliche Salze.

Bevorzugt werden dabei heterocyclische Verbindung, bei denen die Reste in Formel (I) folgende Bedeutungen haben:

- $R^4$ Isobutyl, Benzyl oder Cyclohexylmethyl;
- $R^5$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy;
- $R^6$ ein Rest der Formel (II), worin
  $R^7$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht,
- s 0,1 oder 2, und
- Z für einen Heteroarylrest aus der Gruppe Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, $\beta$-Carbolinyl oder ein benzannelliertes, cyclopenta-, cyclohexa- oder cyclohepta-annelliertes Derivat aus dieser Gruppe steht, und die übrigen Reste wie in Anspruch 1 definiert sind,
  sowie deren physiologisch verträgliche Salze.

Ganz besonders bevorzugt sind Verbindungen der Formel (I), in welcher die Reste folgende Bedeutung haben:

- $R^1$ Methyl, Ethyl, Propyl, i-Propyl, Butyl, i-Butyl oder Pentyl;

$R^2$ Phenyl oder 3-Pyridyl, wobei der Pyridylrest gegebenenfalls einen Methyl- oder Ethyl-Substituenten trägt.
$R^4$ Isobutyl, Benzyl oder Cyclohexylmethyl;
$R^5$ Wasserstoff oder Hydroxy;
$R^6$ ein Rest der Formel (II), worin
$R^7$ für Wasserstoff oder Fluor steht,
Z für einen 2-, 3- oder 4-Pyridinrest, einen 2-, 4- oder 5-Imidazolrest oder einen 2-Oxazolinrest steht, wobei die genannten Heterocyclen jeweils durch einen oder zwei gleiche oder verschiedene Reste aus der Reihe Methyl, Ethyl, Propyl, Allyl, Fluor, Chlor, Brom, $CF_3$ und Methoxy substituiert sein können, und
s 0,1 oder 2 ist;

Die Chiralitätszentren in den Verbindungen der Formel (I) können die R- oder S-Konfiguration aufweisen.

Der Terminus "Aryl" bezeichnet z.B. mono- oder bicyclische Kohlenstoff-Ringsysteme mit einem oder mehreren aromatischen Ringen wie Phenyl, Naphthyl-Tetrahydronaphthyl, Indanyl und dergleichen. Aryl-Reste können dabei unsubstituiert sein oder substituiert durch 1,2 oder 3 Substituenten, die unabhängig voneinander ($C_1$-$C_7$)-Alkyl, Halogenalkyl, Alkoxy, Thioalkoxy, Amino, Alkylamino, Hydroxy, Halogen, Mercapto, Nitro, Carboxaldehyd, Carboxy und Carboxamide sein können.

Halogen ist Fluor, Chlor, Brom oder Jod.

Unter ($C_1$-$C_{10}$)-Alkyl ist ein Alkylrest oder ein entsprechend ungesättigter Rest mit 1-10 C-Atomen zu verstehen. Der Rest kann geradkettig oder verzweigt sein. Entsprechendes gilt für davon abgeleitete Reste, wie z.B. Alkoxy, Alkylthio, Alkylamino, Alkanoyl und Aralkyl.

Unter ($C_3$-$C_8$)-Cycloalkyl werden vorzugsweise Cyclopropyl, Cyclobutyl, Cyclopentyl, Cyclohexyl, Cycloheptyl und Cyclooctyl verstanden. Falls diese Cyclen mehr als einen Substituenten tragen, so können diese sowohl cis als trans zueinander stehen.

Der Rest Z ist beispielsweise ein Heteroarylrest wie Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzaneliertes, cyclopenta-, cyclohexa- oder cyclohepta-aneliertes Derivat dieser Reste. Dieser Heterocyclus kann an einem Stickstoffatom durch Oxido, ($C_1$-$C_6$)-Alkyl, vorzugsweise Methyl oder Ethyl, Phenyl oder Phenyl-($C_1$-$C_4$)-alkyl, z.B. Benzyl, und/oder an einem oder mehreren C-Atomen durch ($C_1$-$C_4$)-Alkyl wie z.B. Methyl, Phenyl, Phenyl-($C_1$-$C_4$)-alkyl wie z.B. Benzyl, Halogen wie z.B. Chlor, Hydroxy, ($C_1$-$C_4$)-Alkoxy wie z.B. Methoxy, Phenyl-($C_1$-$C_4$)-alkoxy wie z.B. Benzyloxy, oder Oxo bis zu trisubstituiert und teilweise gesättigt sein. Vorzugsweise bedeutet Z eine der folgenden Gruppen: 2- oder 3-Pyrrolyl, Phenylpyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-Pyridino, 2-Pyrazinyl, 2-, 4- oder 5-Pyrimidyl, 2-, 3- oder 5-Indolyl, substituiertes 2-Indolyl, z.B. 1-Methyl-, 5-Methyl-, 5-Methoxy, 5-Benzyloxy, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxalolyl, 2-Benzthiazolyl, Benz[e]indol-2-yl oder β-Carbolin-3-yl.

Teilhydrierte oder vollständig hydrierte, heterocyclische Ringe sind beispielsweise Dihydropyridinyl, Pyrrolidinyl, z.B. 2-, 3- oder 4-N-Methylpyrrolidinyl, Piperidinyl, Piperazinyl, Morpholino, Thiomorpholino, Tetrahydrothiophenyl.

Unter Salzen von Verbindungen der Formel (I) sind insbesondere pharmazeutisch verwendbare und nicht-toxische Salze zu verstehen.

Solche Salze werden beispielsweise von Verbindungen der Formel (I), welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin.

Verbindungen der Formel (I), welche basiche Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure oder p-Toluolsulfonsäure.

Die Erfindung betrifft weiterhin ein Verfahren zur Herstellung von Verbindungen der Formel (I), wobei man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel (I) mit einer endständigen Carboxylgruppe besitzen die nachstehende Formel (III)

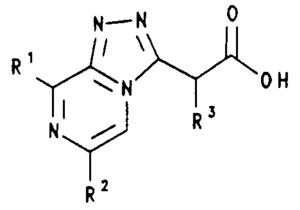

(III)

Fragmente einer Verbindung der Formel (I) mit einer endständigen Aminogruppe besitzen die nachstehende Formel (IV)

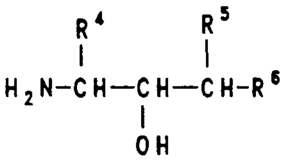

. (IV)

Methoden, die zur Herstellung einer Amidbindung geeignet sind, werden z.B. in Houben-Weyl (Methoden der organischen Chemie, Band 15/2), Bodanszky et al. (Peptide Synthesis, 2nd ed. (Wiley & Sons; New York 1976)) oder Grass, Meienhofer (The Peptides-Analysis, Synthesis, Biology (Academic Press, New York 1979)) beschrieben.

Vorzugsweise werden die folgenden Methoden herangezogen: Aktivestermethode mit N-Hydroxysuccinimid oder 1-Hydroxybenzotriazol als Esterkomponente, Kupplung mit einem Carbodiimid wie Dicyclohexylcarbodiimid oder mit Propanphosphonsäureanhydrid oder Methylethylphosphinsäureanhydrid und die Gemischt-Anhydrid-Methode mit Pivaloylchlorid (M. Zaoral, Coll. Chem. Commun., 1962, 27, 1273). Man führt die Reaktion in einem inerten Lösungsmittel oder Lösungsmittelgemischen vorzugsweise bei einer Temperatur zwischen -20 °C und dem Siedepunkt des Reaktionsgemisches durch.

Die Herstellung der Carbonsäuren der Formel (III) erfolgte nach R. H. Bradbury et al. (EP-A 369 743), D. A. Roberts et al. (J. Med. Chem. 33 (1990), 2326-2334), R. H. Bradbury et al. (J. Med. Chem. 33 (1990), 2335-2342) und R. H. Bradbury et al. (J. Med. Chem. 34 (1991), 151-157).

Die Herstellung der als Ausgangsverbindungen verwendeten optisch aktiven Amine der Formel (IV), worin $R^4$, $R^5$ und $R^6$ wie oben definiert sind, erfolgt ausgehend von optisch aktiven $\alpha$-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppen Aminoalkohole der Formel (IV) ergibt. Bei $R^5$ = OH dient ebenfalls ein N-geschützter Aminosäurealdehyd als Ausgangsmaterial, der z.B. durch aldol-analoge Addition von ungesättigten Verbindungen, Einführungen geeigneter Schutzgruppen und anschließende Epoxidierung in die benötigten Zwischenprodukte umgewandelt wird. Man erhält Diastereomerengemische bezüglich des OH-tragenden Zentrums, die in an sich bekannter Weise, beispielsweise durch fraktionierte Kristallisation oder durch Chromatographie getrennt werden. Die Überprüfung der Diastereomerenreinheit erfolgt beispielsweie mittels HPLC; die Enantiomerenreinheit kann in bekannter Weise durch Überführung in Mosher-Derivate überprüft werden (H. S. Mosher et al., J. Org. Chem. 34 (1969) 2543).

Die Herstellung N-geschützter Aminosäurealdehyde erfolgt nach B. Castro et al. (Synthesis 1983, 676).

Die Aldol-analoge Addition an N-geschützte Aminosäurealdehyde (bevorzugterweise N-tert.-Butyloxycarbonyl- und Benzyloxycarbonyl-Schutzgruppen) erfolgt in einem gegenüber Basen inerten Lösungsmittel, wie Ether, TMF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium, oder Phenyllithium, Natriumamid sowie Alkalimetallsalze von organischen Stickstoffbasen, wie Lithiumdiisopropylamid verwendet werden.

Die zur Herstellung von Verbindungen der Formel (I) erforderlichen Vor- und Nachoperationen wie Einführung und Abspaltung von Schutzgruppen sind literaturbekannt und sind z.B. in T.W. Greene, "Protective Groups Synthesis", beschrieben. Salze von Verbindungen der Formel (I) mit salzbildenden Gruppen werden in an sich bekannter Weise hergestellt, indem man z.B. eine Verbindung der Formel (I) mit einer basischen Gruppe mit einer stöchiometrischen Menge einer geeigneten Säure umsetzt.

Steroisomerengemische, insbesondere Diastereomerengemische, die bei Verwendung racemischer Carbonsäurederivate anfallen, können in an sich bekannter Weise durch fraktionierte Kristallisation oder durch Chromatographie getrennt werden.

Die erfindungsgemäßen Verbindungen der Formel (I) weisen enzymhemmende Eigenschaften auf; insbesondere hemmen sie die Wirkung des natürlichen Enzyms Renin. Renin ist ein proteolytisches Enzym aus der Klasse der Aspartyl-Proteasen, welches als Folge verschiedener Stimuli (Volumendepletion, Natirummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

1. Testprinzip

Beispielsweise wird Humanplasma , welches sowohl Renin als auch Angiotensinogen enthält, bei 37 °C mit der zu testenden Verbindung inkubiert. Dabei wird aus Angiotensiogen unter der Einwirkung von Renin Angiotensin I freigesetzt, das anschließend mit einem handelsüblichen Radioimmunoassay gemessen werden kann. Dies Angiotensin-Freisetzung wird durch Renin-Inhibitoren gehemmt.

2. Gewinnung des Plasmas

Das Blut wird von freiwilligen Probanden gewonnen (ca. 0,5 l pro Person; Bluko-Entnahmegerät (der Fa. ASID Bonz und Sohn, Unterschleißheim)) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4 °C, 15 min; wiederholen, falls erforderlich) wird das Plasma vorsichtig abpipettiert und in geeigneten Portionen bei -30 °C eingefroren. Für den Test werden nur Plasmen mit ausreichend hoher Reninaktivität verwendet. Plasmen mit niedriger Reninaktivität werden durch eine Kältebehandlung (-4 °C, 3 Tage) aktiviert (Prorenin → Renin).

3. Durchführung des Tests

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:

| | |
|---|---|
| Inkubationsansatz: | 1000 µl Plasma (bei 0-4 °C aufgetaut) |
| | 100 µl Phosphatpuffer (pH 7,4) |
| | Zusatz von $10^{-4}$ M Ramiprilat) |
| | 10 µl PMSF-Lösung |
| | 10 µl 0,1 % Genapol PFIC |
| | 12 µl DMSO bzw. Testpräparat |

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37 °C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentration der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockenes eingefroren und bei ca. -25 °C für die Angiotensin I-Bestimmung aufbewahrt (Mittelwert aus drei Einzelproben).

Angiotensin I-Radioimmunoassay (RIA)

Es wird exakt die Gebrauchsanweiseung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel (I) zeigen in dem dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum invivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1-5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel (I) können als Antihypertensiva, sowie zur Behandlung der Herzinsuffizenz verwendet werden.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym deren Inhibition den Lebenszyklus des Virus unterbricht und seine Vermehrung unterbindet.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirkung, die die erfindungsgemäßen Verbindungen insbesondere zur Therapie und Prophylaxe von durch Infektion mit HIV bedingten Erkrankungen qualifiziert. Die erfindungsgemäßen Verbindungen der allgemeinen Formel (I) zeigen in verwendeten in-vitro-Tests Hemmwirkungen bei Konzentrationen von etwa $10^{-4}$ bis $10^{-8}$ Mol/l.

Zum Gegenstand der Erfindung gehört weiterhin die Verwendung von Verbindungen der Formel (I) zur Herstellung von Arzneimittel zur Bluthochdrucktherapie und der Behandlung der kongestiven Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel (I) vorzugsweise zusammen mit einem anorganischem oder organischen pharmazeutischen verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoff, Stabilisatoren oder inerten Verdünnungsmitteln vermischt und durch übliche Methoden in geeignete Darreichungsformen wie Tabletten, Dragees, Steckkapseln, wäßrige, alkoholische oder Ölige Lösungen gebracht. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspension oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Verzeichnis der verwendeten Abkürzungen:

| | |
|---|---|
| Boc | tert.-Butoxycarbonyl |
| BuLi | n-Butyl-Lithium |
| DCC | Dicyclohexylcarbodiimid |
| DCI | Desorption Chemical Ionisation |

| | |
|---|---|
| DNP | 2,4-Dinitrophenyl |
| DME | Dimethoxyethan |
| DMF | Dimethylformamid |
| EE | Essigsäureethylester |
| FAB | Fast atom bombardment |
| h | Stunde |
| HOBt | 1-Hydroxybenzotriazol |
| M | Molekularpeak |
| MS | Massenspektrum |
| min | Minuten |
| NEM | N-Ethylmorpholin |
| RT | Raumtemperatur |
| THF | Tetrahydrofuran |
| Trt | Triphenylmethyl |

Die folgenden Beispiele verdeutlichen die Herstellung der erfindungsgemäßen Verbindungen.

Beispiel 1

N-[(8-Isobutyl-6-phenyl-1,2,4-triazolo[4,3-a]pyrazin-3-yl]-ethanoyl]-(2S,3R,4S)-1-cyclohexyl-3,4-dihydroxy-6-(2-pyridyl)-2-hexylamin

1a) (2S,3R,4S)-2-(tert.-Butyloxycarbonyl)amino-1-cyclohexyl-3,4-dihydroxy-6-(2-pyridyl)-hexan

186 mg 2-Picolin in 20 ml TMF werden bei -78 °C mit 2,8 ml n-Butyllithium versetzt. Nach Aufwärmen auf Raumtemperatur wird 30 Min. gerührt, dann auf -40 °C abgekühlt. 2 mmol (2RS,3R,4S)-3-tert.-Butyldimethyl-silyloxy-4-(tert.-butyloxycarbonyl)amino-5-cyclohexyl-1,2-oxopentan (bekannt aus EP-A 189 203, Beispiel 6) werden zugegeben (gelöst in 10 ml THF). Nach 10 h Rühren bei Raumtemperatur wird mit Wasser verdünnt und mit Methyl-tert.-butylether extrahiert. Das Rohprodukt wird nach Einengen in THF gelöst und mit 10 ml einer 1 M Lösung von Tetrabutylammoniumfluorid in THF 1 h bei 0 °C gerührt. Nach Verdünnen mit Wasser, Extrahieren mit EE und Einengen erhält man 300 mg des (2S,3R,4S)-Isomeren (MS (FAB): 393 (M+M)) und 240 mg des (2S,3S,4S)-Isomeren (MS (FAB): 393 (M+M) nach chromatographischer Trennung an Kieselgel.

1b) N-[(8-Isobutyl-6-phenyl-1,2,4-triazolo[4,3-a]pyrazin-3-yl)-ethanoyl]-(2S,3R,4S)-1-cyclohexyl-3,4-dihydroxy-6(2-pyridyl)-2-hexylamin

400 mg (2S,3R,4S)-2-(tert.-Butyloxycarbonyl)amino-1-cyclohexyl-3,4-dihydroxy-6(2-pyridyl)-hexan (Verbindung aus Beispiel 1a) werden in 5 ml abs. $CH_2Cl_2$ gelöst, 5 ml Trifluoressigsäure zugesetzt und die resultierende Lösung 3 h bei Raumtemperatur gerührt. Der nach Einengung erhaltene Rückstand wird in 1 M $NaHCO_3$-Lösung aufgenommen, das Gemisch dreimal mit EE extrahiert und die vereinten EE-Phasen über $Na_2SO_4$ getrocknet. Nach Einengung wird der Rückstand in 5 ml abs. DMF gelöst, 316,6 mg (8-Isobutyl-6-phenyl-1,2,4-triazolo[4,3-a]pyrazin-3-yl)-essigsäure (bekannt aus EP-A 369 743, Beispiel 1), 208,4 mg N,N'-Dicyclohexylcarbodiimid und 206,8 mg 1-Hydroxybenzotriazol hinzugegeben, der pH der Lösung mit N-Ethylmorpholin auf 9 eingestellt und 50 h bei Raumtemperatur gerührt. Nach Filtration wird mit EE verdünnt und je einmal mit gesättigter $NaHCO_3$-Lösung, Wasser und gesättigter Kochsalzlösung gewaschen, mit einem $CH_2Cl_2$ (MCOH-Laufmittelgemisch ergibt 207,4 mg der Titelverbindung.
MS(FAB):585 (M+H)

Beispiel 2

N-[2-(8-Isobutyl-6-phenyl-1,2,4-triazolo[4,3-a]pyrazin-3-yl)-3-(3-pyridyl)propionyl]-(2S,3R,4S)-1-cyclohexyl-3,4-dihydroxy-6(2-pyridyl)-2-hexylamin

Die Titelverbindung resultiert aus der Umsetzung von 2-[8-Isobutyl-6-phenyl-1,2,4-triazolo[4,3-a]pyrazin-3-yl]-3-(3-pyridyl)-propionsäure (bekannt aus EP-A 369 743, Beispiel 3) mit der Verbindung aus Beispiel 1a nach dem Verfahren des Beispiels 1b.
MS (FAB): 676 (M+H)

Beispiel 3

N-[(8-Propyl-6(3-pyridyl)-1,2,4-triazolo[4,3-a]pyrazin-3-yl)-ethanoyl]-(2S,3R,4S)-1-cyclohexyl-3,4-dihydroxy-6-(2-pyridyl)-2-hexylamin

Die Titelverbindung wird aus (8-Propyl-6-(3-pyridyl)-1,2,4-triazolo[4,3-1]pyrazin-3-yl-essigsäure (bekannt aus EP-A 369 743, Beispiel 4) und der Verbindung aus Beispiel 1a nach dem in Beispiel 1b angeführten Verfahren hergestellt.
MS (FAB): 572 (M + H)

Beispiel 4

N-[2-(8-Propyl-6-(3-pyridyl)-1,2,4-triazolo[4,3-a]pyrazin-3-yl]-3-(3-pyridyl)-propionyl]-(2S,3R,4S)-1-cyclohexyl-3,4-dihydroxy-6(2-pyridyl)-2-hexylamin

Die Titelverbindung wird aus 2-[8-Propyl-6-(3-pyridyl)-1,2,4-triazolo[4,3-a]pyrazin-3-yl]-3-(3-pyridyl)-propionsäure (bekannt aus EP-A 369 743, Beispiel 6) und der Verbindung aus Beispiel 1a nach dem Verfahren des Beispiels 1b dargestellt.
MS (FAB): 663 (M + H)

Beispiel 5

N-[(3-(Imidazol-4-yl)-(2RS)-2-(8-propyl-6-(3-pyridyl)-1,2,4-triazolo(4,3-a]pyrazin-3-yl)-propionyl]-(2S,3R,4S)-1-cyclohexyl-3,4-dihydroxy-6-(2-pyridyl)-2-hexylamin

Die Titelverbindung wird aus 3-(1-Triphenylmethylimidazol-4-yl)-2-[8-propyl-6-(3-pyridyl)-1,2,4-triazolo-[4,3-a]pyrazin-3-yl]-propionsäure (bekannt aus EP-A 369 743, Beispiel 27) und der Verbindung aus Beispiel 1a, nach dem Verfahren des Beispiels 1b und anschließender $N^{im}$-Trt-Schutzgruppenabspaltung mittels 90 %iger Trifluoressigsäure und extraktiver Aufarbeitung hergestellt.
MS (FAB): 652 (M + H)

**Patentansprüche**

**1.** Heterocyclische Verbindung der Formel (I),

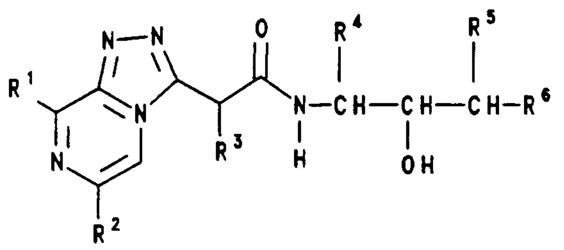

(I)

in welcher die Reste folgende Bedeutungen haben:

- $R^1$ ($C_1$-$C_8$)-Alkyl oder Phenyl;
- $R^2$ Phenyl oder Pyridyl, wobei der Pyridylrest gegebenenfalls einen ($C_1$-$C_4$)-Alkylsubstituenten trägt;
- $R^3$ Wasserstoff oder eine Gruppe der Formel Q-A, in welcher Q steht für Pyridyl, Imidazolyl, Thiazolyl und Pyrazolyl, und A Methylen oder Ethylen ist;
- $R^4$ Wasserstoff, ($C_1$-$C_{10}$)-Alkyl, ($C_3$-$C_8$)-Cycloalkyl, ($C_3$-$C_8$)-Cycloalkyl-($C_1$-$C_4$)-alkyl, ($C_6$-$C_{12}$)-Aryl oder ($C_6$-$C_{12}$)-Aryl-($C_1$-$C_4$)-alkyl;
- $R^5$ Wasserstoff, ($C_1$-$C_{10}$)-Alkyl, ($C_6$-$C_{12}$)-Aryl, ($C_6$-$C_{12}$)-Aryl-($C_1$-$C_4$)-alkyl, Hydroxy oder Amino;
- $R^6$ ein Rest der Formel (II),

$(CH_2)_s$-$CHR^7$-Z (II)

wobei

$R^7$ für Wasserstoff, $(C_1-C_7)$-Alkyl, $(C_1-C_4)$-Alkoxy, $(C_1-C_4)$-Alkylthio, $(C_1-C_4)$-Alkylamino, Hydroxy, Azido oder Halogen steht, und

Z für einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann, und der durch einen oder zwei gleiche oder verschiedene Reste aus der Reihe $(C_1-C_4)$-Alkyl, Allyl, $(C_1-C_4)$-Alkoxy, Hydroxy, Halogen, Amino, Mono- oder Di-$(C_1-C_4)$-alkylamino und $CF_3$ substituiert sein kann; und wobei

s 0, 1, 2, 3 oder 4 bedeutet

sowie deren physiologisch verträgliche Salze.

**2.** Heterocyclische Verbindung gemäß Anspruch 1, dadurch gekennzeichnet, daß die Reste in Formel (I) folgende Bedeutungen haben:

$R^4$ Isobutyl, Benzyl oder Cyclohexylmethyl;

$R^5$ Wasserstoff, $(C_1-C_5)$-Alkyl, $(C_6-C_{10})$-Aryl, $(C_6-C_{10})$-Aryl-$(C_1-C_4)$-alkyl oder Hydroxy;

$R^6$ ein Rest der Formel (II), worin

$R^7$ für Wasserstoff, $(C_1-C_4)$-Alkyl, $(C_1-C_4)$-Alkoxy, $(C_1-C_4)$-Alkylthio, $(C_1-C_4)$-Alkylamino, Hydroxy, Azido oder Halogen steht,

s 0,1 oder 2, und

Z für einen Heteroarylrest aus der Gruppe Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, $\beta$-Carbolinyl oder ein benzannelliertes, cyclopenta-, cyclohexa- oder cyclohepta-annelliertes Derivat aus dieser Gruppe steht, und die übrigen Reste wie in Anspruch 1 definiert sind,

sowie deren physiologisch verträgliche Salze.

**3.** Heterocyclische Verbindung gemäß Anspruch 1, dadurch gekennzeichnet, daß die Reste in Formel (I) folgende Bedeutungen haben:

$R^1$ Methyl, Ethyl, Propyl, i-Propyl, Butyl, i-Butyl oder Pentyl;

$R^2$ Phenyl oder 3-Pyridyl, wobei der Pyridylrest gegebenenfalls einen Methyl- oder Ethyl-Substituenten trägt,

$R^4$ Isobutyl, Benzyl oder Cyclohexylmethyl;

$R^5$ Wasserstoff oder Hydroxy;

$R^6$ ein Rest der Formel (II), worin

$R^7$ für Wasserstoff oder Fluor steht,

Z für einen 2-, 3- oder 4-Pyridinrest, einen 2-, 4- oder 5-Imidazolrest oder einen 2-Oxazolinrest steht, wobei die genannten Heterocyclen jeweils durch einen oder zwei gleiche oder verschiedene Reste aus der Reihe Methyl, Ethyl, Propyl, Allyl, Fluor, Chlor, Brom, $CF_3$ und Methoxy substituiert sein können, und s 0,1 oder 2 ist;

und die übrigen Reste wie in Anspruch 1 definiert sind, sowie deren physiologisch verträgliche Salze.

**4.** Verfahren zur Herstellung einer heterocyclischen Verbindung der Formel (I) gemäß Anspruch 1, wobei man ein Fragment der Formel (III) mit endständiger Carboxylgruppe oder dessen reaktives Derivat mit einem Fragment der Formel (IV) mit freier Aminogruppe kuppelt,

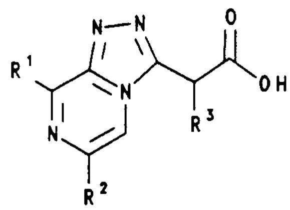

(III)

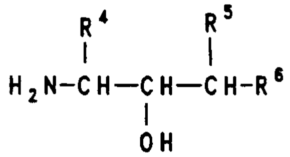

(IV)

gegebenenfalls zum Schutz weiterer funktioneller Gruppen (eine) temporär eingeführte Schutzgruppe(n) abspaltet und die so erhaltene Verbindung gegebenenfalls in ein physiologisch verträgliches Salz überführt.

**5.** Heterocyclische Verbindung der Formel (I) gemäß einem der Ansprüche 1 bis 3 zur Anwendung als Heilmittel.

**6.** Heterocyclische Verbindung der Formel (I) gemäß einem der Ansprüche 1 bis 3 zur Anwendung als Heilmittel bei der Behandlung des Bluthochdrucks.

**7.** Pharmazeutische Zubereitung enthaltend einen Wirkstoff und gegebenenfalls Trägerstoffe und weitere Zusätze, dadurch gekennzeichnet, daß sie als Wirkstoff mindestens eine Verbindung der Formel (I) gemäß Anspruch 1 enthält.

**8.** Verfahren zur Herstellung einer pharmazeutischen Zubereitung gemäß Anspruch 7, dadurch gekennzeichnet, daß man den oder die Wirkstoffe zusammen mit einem physiologisch unbedenklichen Träger und gegebenenfalls weiteren Zusätzen in eine geeignete Darreichungsform bringt.

**Patentansprüche für folgenden Vertragsstaaten : ES, GR**

**1.** Verfahren zur Herstellung einer heterocyclischen Verbindung der Formel (I),

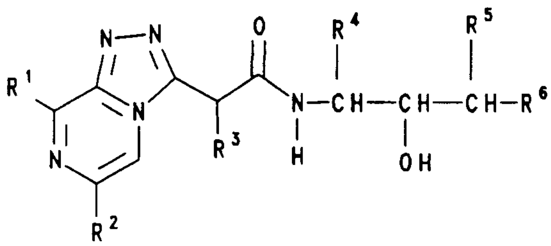

(I)

in welcher die Reste folgende Bedeutungen haben:

$R^1$ $(C_1\text{-}C_8)$-Alkyl oder Phenyl;

$R^2$ Phenyl oder Pyridyl, wobei der Pyridylrest gegebenenfalls einen $(C_1\text{-}C_4)$-Alkylsubstituenten trägt;

$R^3$ Wasserstoff oder eine Gruppe der Formel Q-A, in welcher Q steht für Pyridyl, Imidazolyl, Thiazolyl und Pyrazolyl, und A Methylen oder Ethylen ist;

$R^4$ Wasserstoff, $(C_1\text{-}C_{10})$-Alkyl, $(C_3\text{-}C_8)$-Cycloalkyl, $(C_3\text{-}C_8)$-Cycloalkyl-$(C_1\text{-}C_4)$-alkyl, $(C_6\text{-}C_{12})$-Aryl oder $(C_6\text{-}C_{12})$-Aryl-$(C_1\text{-}C_4)$-alkyl;

$R^5$ Wasserstoff, $(C_1\text{-}C_{10})$-Alkyl, $(C_6\text{-}C_{12})$-Aryl, $(C_6\text{-}C_{12})$-Aryl-$(C_1\text{-}C_4)$-alkyl, Hydroxy oder Amino;

$R^6$ ein Rest der Formel (II),

$(CH_2)_s$-$CHR^7$-Z (II)

wobei

$R^7$ für Wasserstoff, ($C_1$-$C_7$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht, und

Z für einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann, und der durch einen oder zwei gleiche oder verschiedene Reste aus der Reihe ($C_1$-$C_4$)-Alkyl, Allyl, ($C_1$-$C_4$)-Alkoxy, Hydroxy, Halogen, Amino, Mono- oder Di-($C_1$-$C_4$)-alkylamino und $CF_3$ substituiert sein kann; und wobei

s 0,1,2,3 oder 4 bedeutet

sowie deren physiologisch verträglichen Salze, dadurch gekennzeichnet, daß man ein Fragment der Formel (III) mit endständiger Carboxylgruppe oder dessen reaktives Derivat mit einem Fragment der Formel (IV) mit freier Aminogruppe kuppelt,

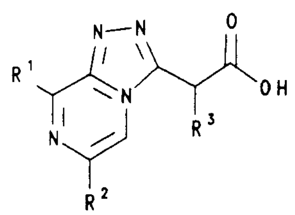

(III)

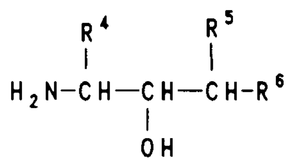

(IV)

gegebenenfalls zum Schutz weiterer funktioneller Gruppen (eine) temporär eingeführte Schutzgruppe(n) abspaltet und die so erhaltene Verbindung gegebenenfalls in ein physiologisch verträgliches Salz überführt.

2. Verfahren gemäß Anspruch 1, dadurch gekennzeichnet, daß die Reste in Formel (I) folgende Bedeutungen haben:
   - $R^4$ Isobutyl, Benzyl oder Cyclohexylmethyl;
   - $R^5$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy;
   - $R^6$ ein Rest der Formel (II), worin
     $R^7$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht,
   - s 0,1 oder 2, und
   - Z für einen Heteroarylrest aus der Gruppe Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzannelliertes, cyclopenta-, cyclohexa- oder cyclohepta-annelliertes Derivat aus dieser Gruppe steht, und die übrigen Reste wie in Anspruch 1 definiert sind.

3. Verfahren gemäß Anspruch 1, dadurch gekennzeichnet, daß die Reste in Formel (I) folgende Bedeutungen haben:
   - $R^1$ Methyl, Ethyl, Propyl, i-Propyl, Butyl, i-Butyl oder Pentyl;
   - $R^2$ Phenyl oder 3-Pyridyl, wobei der Pyridylrest gegebenenfalls einen Methyl- oder Ethyl-Substituenten trägt,
   - $R^4$ Isobutyl, Benzyl oder Cyclohexylmethyl;
   - $R^5$ Wasserstoff oder Hydroxy;

$R^6$ ein Rest der Formel (II), worin $R^7$ für Wasserstoff oder Fluor steht, Z für einen 2-, 3- oder 4-Pyridinrest, einen 2-, 4- oder 5-Imidazolrest oder einen 2-Oxazolinrest steht, wobei die genannten Heterocyclen jeweils durch einen oder zwei gleiche oder verschiedene Reste aus der Reihe Methyl, Ethyl, Propyl, Allyl, Fluor, Chlor, Brom, $CF_3$ und Methoxy substituiert sein können, und s 0,1 oder 2 ist;

und die übrigen Reste wie in Anspruch 1 definiert sind.

**4.** Verwendung einer heterocyclischen Verbindung der Formel (I) gemäß Anspruch 1 als Heilmittel.

**5.** Verwendung einer heterocyclischen Verbindung der Formel (I) gemäß Anspruch 1 als Heilmittel bei der Behandlung des Bluthochdrucks.

**6.** Verfahren zur Herstellung einer pharmazeutischen Zubereitung enthaltend einen Wirkstoff hergestellt gemäß Anspruch 1, dadurch gekennzeichnet, daß man den oder die Wirkstoffe zusammen mit einem physiologisch unbedenklichen Träger und gegebenenfalls weiteren Zusätzen in eine geeignete Darreichungsform bringt.